(19) Europäisches Patentamt
European Patent Office
Office européen des brevets

(11) Veröffentlichungsnummer: **0 180 890**
**B1**

(12) # EUROPÄISCHE PATENTSCHRIFT

(45) Veröffentlichungstag der Patentschrift: 04.01.89

(51) Int. Cl.⁴: C 07 D 313/08

(21) Anmeldenummer: 85113703.4

(22) Anmeldetag: 28.10.85

**E R R A T U M**

| DIE TEXTSTELLE: TEXT PUBLISHED: LE PASSAGE SUIVANT: | (SEITE, SPALTE, ZEILE) (PAGE, COLUMN, LINE) (PAGE, COLONNE, LIGNE) | | LAUTET BERICHTIGT: SHOULD READ: DEVRAIT ETRE LU: |
|---|---|---|---|
| der Hydrochlorid-Gemisches | 6 | 1 | der Mengenanteil der einzelnen Komponenten des analysierten Hydrochlorid-Gemisches |

Tag der Entscheidung über die Berichtigung / Date of decision on rectification: / Date de décision portant sur modification: 24.2.89

Ausgabe- und Veröffentlichungstag: / Issue and publication date: / Date d'edition et de publication: 19.4.89

Patbl.Nr / EPB no: / Bull. no: 89/16

(19) Europäisches Patentamt
European Patent Office
Office européen des brevets

(11) Veröffentlichungsnummer: **0 180 890**
**B1**

# (12) EUROPÄISCHE PATENTSCHRIFT

(45) Veröffentlichungstag der Patentschrift: **04.01.89**

(51) Int. Cl.⁴: **C 07 D 313/08**

(21) Anmeldenummer: **85113703.4**

(22) Anmeldetag: **28.10.85**

(54) **Verfahren zur diastereoselektiven Reduktion von 3-Amino-1-benzoxepin-5(2H)-onen.**

(30) Priorität: **05.11.84 DE 3440295**

(43) Veröffentlichungstag der Anmeldung: **14.05.86 Patentblatt 86/20**

(45) Bekanntmachung des Hinweises auf die Patenterteilung: **04.01.89 Patentblatt 89/1**

(84) Benannte Vertragsstaaten: **AT BE CH DE FR GB IT LI NL SE**

(56) Entgegenhaltungen:
**EP-A-0 024 560**

**JOURNAL OF THE AMERICAN CHEMICAL SOCIETY, Band 78, 1956, Seiten 140-150, Washington, US; A.P. PHILLIPS et al.: "Catalytic and chemical hydrogenations of 2-benzylidene- and 2-benzylcyclopentanones. Cis- and Trans-2-benzylcyclopentanols and some derivatives"**
**JOURNAL OF THE AMERICAN CHEMICAL SOCIETY, Band 74, 1952, Seiten 249-251, Washington, US; N.F. ALBERTSON: "So-called 5-carbethoxy-9-methyl-2-oxo-1-aza-bicyclo 3.3.1 nonane. A correction"**

(73) Patentinhaber: **Kali- Chemie Pharma GmbH, Hans-Böckler- Allee 20 Postfach 220, D-3000 Hannover 1 (DE)**

(72) Erfinder: **Ohlendorf, Heinrich Wilhelm, Dr. Dipl.-Chem., Schuhmachersweg 18, D-3008 Garbsen 2 (DE)**
Erfinder: **Mätzel, Uwe, Dr. Dipl.- Chem., Habichtshorst 9, D-3167 Burgdorf (DE)**

(74) Vertreter: **Lauer, Dieter, Dr., c/o Kali- Chemie AG Postfach 220 Hans- Böckler- Allee 20, D-3000 Hannover 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 180 890 B1

LIBER, STOCKHOLM 1989

**Beschreibung**

Verfahren zur diastereoselektiven Reduktion von 3-Amino-1-benzoxepin-5(2H)-onen.

Die vorliegende Erfindung betrifft ein Verfahren zur diastereoselektiven Reduktion von 3-Amino-1-benzoxepin-5(2H)-onen zu 2,3,4,5-Tetrahydro-3-amino-1-benzoxepin-5-olen, in welchen die Substituenten in 3- und 5-Stellung vorwiegend cis-ständig zueinander sind.

2,3,4,5-Tetrahydro-3-amino-1-benzoxepin-5-ole und deren Säureadditionssalze sind aus der europäischen Patentanmeldung Nr. 24560 bekannt. Die Verbindungen besitzen 2 chirale Zentren ($C_5$ und $C_3$, vgl. nachstehende Formel I), deren Substituenten jeweils in R- oder S-Stellung angeordnet sein können, so daß die Substanzen in mehreren stereoisomeren Formen auftreten. Die Substanzen besitzen auf die gastrointestinale Motilität günstig wirkende pharmakologische Eigenschaften und sind daher als Pharmazeutica verwendbar. Hierbei haben sich cis-2,3,4,5-Tetrahydro-3-ami-no-1-benzoxepin-5-ole, worin die Substituenten in 3- und 5-Stellung zueinander cis-ständig sind, für orale Applikation als besonders geeignet erwiesen.

Gemäß den in der europäischen Patentanmeldung beschriebenen Verfahren können die 2,3,4,5-Tetrahydro-3-amino-1-benzoxepin-5-ole aus den entsprechenden 3-Amino-1-benzoxepin-5(2H)-onen durch einstufige oder zweistufige Reduktion durch Behandeln mit bestimmten Hydridreduktionsmitteln und/oder katalytische Hydrierung in Gegenwart von Raney-Nickel hergestellt werden.

Die einstufige Reduktion wird mit Natriumborhydrid in neutralem bis schwach saurem Medium oder mit Natriumcyanoborhydrid in saurem Medium oder durch katalytische Hydrierung in Gegenwart von Raney-Nickel in einem protischen Lösungsmittel erreicht. Hierbei fallen Gemische der verschiedenen diastereoisomeren Formen an.

Aus den Gemischen müssen die Racemate mit cis-Konfiguration bzw. Racemate mit trans-Konfiguration durch aufwendige Trennverfahren z. B. fraktionierte Kristallisation geeigneter Salze oder chromatographische Verfahren, zunächst angereichert und schließlich isoliert werden. Die erhaltenen racemischen cis-2,3,4,5-Tetrahydro-3-amino-1-benzoxepin-5-ole welche weitgehend frei von trans-Epimeren sind, bzw. die entsprechenden Racemate mit trans-Konfiguration, welche weitgehend von cis-Epimeren befreit sind, können anschließend gewünschtenfalls in an sich bekannter Weise in ihre optischen Isomeren aufgetrennt werden.

Bei den in der europäischen Patentanmeldung beschriebenen zweistufigen Reduktionsverfahren werden die 3-Amino-1-benzoxepin-5(2H)-one zunächst durch Hydrierung der 3,4-Doppelbindung in die entsprechenden 3-Amino-3,4-dihydro-1-benzoxepin-5(2H)-one überführt. Diese Reaktion kann durch katalytische Hydrierung in Gegenwart von Raney-Nickel in einem aprotischen Lösungsmittel oder durch Umsetzung mit Natriumcyanoborhydrid in schwach saurem Medium erfolgen. In einer zweiten Reduktionsstufe wird dann die 5-Keto-Gruppe zur Hydroxygruppe reduziert. Diese Reduktion kann mit den in der europäischen Patentanmeldung angegebenen Hydridreduktionsmitteln erfolgen. Nur in diesem zueistufigen Reduktionsverfahren kann in der zweiten Reduktionsstufe bei Verwendung eines speziellen selektiven, jedoch technisch aufwendig zu handhabenden Hydridreduktionsmittels eine Anreicherung von cis-2,3,4,5-Tetrahydro-3-amino-1-benzoxepin-5-olen in dem Reduktionsprodukt erzielt werden.

Der vorliegenden Erfindung liegt die Aufgabe zugrunde, verbesserte Verfahren zur diastereoselektiven Herstellung von cis-2,3,4,5-Tetrahydro-3-amino-1-benzoxepin-5-olen zu entwickeln.

Es wurden nun Verfahren gefunden, mit welchen eine diastereoselektive Reduktion von 3-Amino-1-benzoxepin-5(2H)-onen in einer einzigen Reaktionsstufe in guten Ausbeuten und mit hoher Diastereoselektivität zu 2,3,4,5-Tetrahydro-3-amino-1-benzoxepin-5-olen mit überwiegend cis-Konfiguration erzielt wird.

Gegenstand der Erfindung ist daher ein Verfahren zur Herstellung von cis-2,3,4,5-Tetrahydro-3-amino-1-benzoxepin-5-olen der allgemeinen Formel I

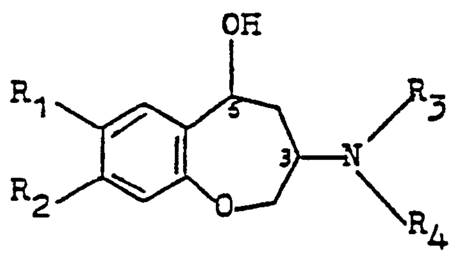

I

worin die Substituenten in 3- und 5-Stellung zueinander cis-ständig sind und

$R_1$ Wasserstoff, Halogen oder Alkyl mit 1-4 Kohlenstoffatomen bedeutet,
$R_2$ Wasserstoff, Halogen oder Alkyl mit 1-4 Kohlenstoffatomen bedeutet,
$R_3$ Wasserstoff oder Alkyl mit 1-5 Kohlenstoffatomen bedeutet, und
$R_4$ Wasserstoff oder Alkyl mit 1-5 Kohlenstoffatomen bedeutet,

und deren Säureadditionssalzen, dadurch gekennzeichnet, daß man 3-Amino-1-benzoxepin-5-(2H)-one der allgemeinen Formel II

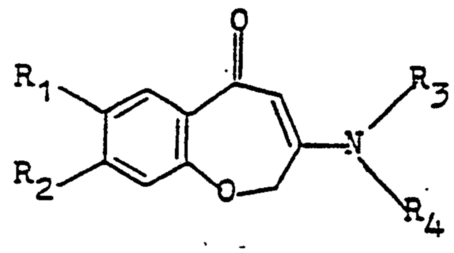

II

worin $R_1$, $R_2$ $R_3$ und $R_4$ obige Bedeutung besitzen, einstufig diastereoselektiv zu einem Gemisch von 2,3,4,5-Tetrahydro-3-amino-1-benzoxepin-5-olen mit einem überwiegenden Anteil an cis-Verbindungen der allgemeinen Formel I reduziert, indem man

a) Verbindungen der allgemeinen Formel II mit einem Boran/ Amin-Komplex in einem 0-50 Vol.-% einer flüssigen, niederen aliphatischen Carbonsäure enthaltenden, aprotischen organischen Lösungsmittel umsetzt, oder

b) Verbindungen der allgemeinen Formel IIa

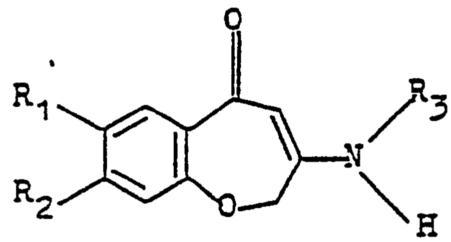

IIa

worin $R_1$, $R_2$ und $R_3$ obige Bedeutung besitzen, mit Wasserstoff in Gegenwart eines Platinoxidkatalysators in einem organischen, protischen, polaren Lösungsmittel in Gegenwart einer äquivalenten Menge einer unter den Reaktionsbedingungen stabilen Säure hydriert,

und aus dem erhaltenen Reduktionsprodukt die cis-2,3,4,5-Tetrahydro-3-amino-1-benzoxepin-5-ole in Form ihrer Säureadditionssalze oder der freien Basen isoliert und gewünschtenfalls die Säureadditionssalze in die freien Basen überführt oder die freien Basen in ihre Säureadditionssalze überführt.

Nach der Verfahrensvariante a) des erfindungsgemäßen Verfahrens werden die 3-Amino-1-benzoxepin-5(2H)-one der Formel II durch Umsetzung mit einem Boran/Amin-Komplex, worin Boran komplex an eine Aminkomponente gebunden ist, reduziert. Verbindungen der Formel II, worin $R_1$ oder $R_2$ Halogen bedeuten, werden bevorzugt nach dieser Verfahrensvariante reduziert. Als Aminkomponenten des Boran/Amin-Komplexes sind insbesondere solche Amine geeignet, welche später leicht aus dem Reaktionsgemisch bzw. von den erhaltenen, ebenfalls basisch reagierenden Verbindungen der Formel I abtrennbar sind. So eignen sich beispielsweise Amine, welche genügend leicht flüchtig sind, so daß sie durch Destillation oder einfaches Abdampfen entfernt werden können. Geeignete Amine sind beispielsweise Amine der Formel III

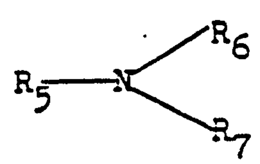

III

worin $R_5$ Wasserstoff, Methyl oder Äthyl bedeutet, $R_6$ Wasserstoff, Methyl oder Äthyl bedeutet, und $R_7$ Wasserstoff, geradkettiges oder verzweigtes niederes Alkyl mit vorzugsweise bis zu 4 Kohlenstoffatomen oder, falls $R_5$ und $R_6$ Wasserstoff sind, auch Phenyl oder durch niederes Alkyl substituiertes Phenyl bedeutet. Geeignete Amine der Formel III sind insbesondere aliphatische Amine, vorzugsweise primäre oder sekundäre Amine, d.h. Amine, worin $R_5$ und/oder $R_6$ Wasserstoff bedeuten und $R_7$ geradkettiges oder verzweigtes Alkyl darstellt. Als Beispiele geeigneter Amine seien genannt tert. Butylamin, Diäthylamin oder Di- oder Tri-Methylamin. Ferner eignen sich auch cyclische Amine wie beispielsweise Pyridin, Morpholin oder N-Niederalkylmorpholin. Als besonders günstig hat sich der Boran/ tert. Butylamin-Komplex erwiesen.

Es werden im allgemeinen mindestens äquimolare Mengen des Boran/Amin-Komplexes eingesetzt. Als zweckmäßig erweisen sich beispielsweise Mengen von 2-3 Mol Boran/Amin-Komplex pro Mol Verbindung der Formel II.

Als Lösungsmittel eignen sich unter den Reaktionsbedingungen stabile organische aprotische, unpolare Lösungsmittel, vorzugsweise solche Lösungsmittel, welche mit flüssigen niedrigen aliphatischen Carbonsäuren mischbar sind, bzw. Gemische dieser unpolaren Lösungsmittel mit niederen aliphatischen Carbonsäuren. Als unpolare aprotische Lösungsmittel eignen sich insbesondere aromatische Kohlenwasserstoffe wie Toluol oder Benzol oder halogenierte Kohlenwasserstoffe wie Methylenchlorid. Als niedere aliphatische Carbonsäuren

eignen sich unter den Reaktionsbedingungen stabile flüssige niedere Alkancarbonsäuren, beispielsweise Alkancarbonsäuren mit 2-4, insbesondere 2-3, Kohlenstoffatomen, vorzugsweise Essigsäure.

Das Lösungsmittel kann bis zu 50 Vol-% der aliphatischen Carbonsäure enthalten. Geeignet sind beispielsweise Lösungsmittelgemische, worin das Vol.-Verhältnis von unpolarem Lösungsmittel zu niederer Carbonsäure zwischen 9 : 1 und 1 : 1, vorzugsweise zwischen 3 : 1 und 1 : 1, insbesondere ca. 2 : 1, beträgt. Als besonders geeignet erweist sich ein Lösungsmittelgemisch, welches Toluol und Eisessig in einem Vol.-Verhältnis von 1,5-2,5 : 1 enthält.

Die Reaktionstemperatur kann zwischen Raumtemperatur und ca. 100 °C betragen und je nach Säuremenge in dem Reaktionsgemisch variieren. So erweisen sich bei einem Verhältnis von unpolarem Lösungsmittel zu aliphatischer Carbonsäure von 3 : 1 bis 1 : 1 Temperaturen zwischen 30 und 80°C, insbesondere 40-60°C, als geeignet, während bei geringerem Säuregehalt bis säurefreien Lösungsmitteln höhere Temperaturen, beispielsweise Temperaturen zwischen 60 und 100, insbesondere 80 und 100°C, zweckmäßiger sind. Die Reaktionszeit variiert je nach den verwendeten Reaktionsbedingungen und kann beispielsweise zwischen ca. 2 und 75 Stunden betragen.

Im Gegensatz zu den aus der europäischen Patentanmeldung Nr. 24560 bekannten Reduktionen mit den dort angegebenen Hydridreduktionsmitteln führt die erfindungsgemäße Reduktion der Verbindungen der Formel II mit Boran/Amin-Komplexen überraschenderweise in nur einem Reaktionsschritt zu hohen Ausbeuten an 2,3,4,5-Tetrahydro-3-amino-1-benzox-epin-5-olen mit einem bereits so hohen Gehalt an cis-Epimeren, daß hieraus weitgehend transepimerenfreie cis-2,3,4,5-Tetrahydro-3-amino-1-benzoxepin-5-ole mit nur geringem Aufwand isoliert werden können. Das erfindungsgemäße Reduktionsverfahren führt nicht nur zu wesentlich verbesserten Ausbeuten an den gewünschten cis-epimeren Verbindungen der Formel I, sondern hat darüber hinaus noch den Vorteil, daß die Handhabung des verwendeten Reduktionsmittels im technischen Maßstab wesentlich einfacher ist als das Arbeiten mit dem einzigen der in der europäischen Patentanmeldung vorgeschlagenen Hydridreduktionsmittel, mit welchem in der zweiten Reaktionsstufe eine Anreicherung an cis-Epimeren in dem Reaktionsprodukt erreicht werden konnte.

Die Hydrierung von Verbindungen der Formel IIa gemäß Verfahrensvariante b) mit Wasserstoff unter Verwendung von Platinoxid als Katalysator erfolgt in organischen, protischen polaren Lösungsmitteln in Gegenwart einer äquivalenten Menge Säure. Als protische, polare, organische Lösungsmittel eignen sich insbesondere niedere Alkohole, beispielsweise Alkohole mit 1-3 Kohlenstoffatomen, bevorzugt Methanol. Zweckmäßigerweise wird praktisch wasserfreies Lösungsmittel verwendet, d. h. das Lösungsmittel sollte vorteilhaft nicht mehr als ca. 1 %, vorzugsweise unter 0,5 %, Wasser enthalten.

Als Säuren eignen sich unter den Reaktionsbedingungen stabile anorganische oder organische Säuren. Beispiele geeigneter anorganischen Säuren sind Halogenwasserstoffsäuren wie Chlorwasserstoffsäure, Schwefelsäure oder Phosphorsäure. Beispiele geeigneter organischer Säuren sind unter den Reaktionsbedingungen nicht hydrierbare, niedere aliphatische Mono- oder Dicarbonsäuren, insbesondere aliphatische Carbonsäuren mit bis zu 5 Kohlenstoffatomen wie beispielsweise Oxalsäure, aromatische Säuren, z. B. Benzolsulfonsäuren, welche gegebenenfalls im Benzolring durch niederes Alkyl oder Halogen substituiert sind, wie beispielsweise p-Toluolsulfonsäure. Zur Erzielung optimaler Ergebnisse ist es wesentlich, daß die Säuremenge so genau dosiert ist, daß im Reaktionsgemisch nur ein Äquivalent Säure ± 5 % pro Mol der Verbindung der Formel IIa vorliegt.

Gewünschtenfalls können die Verbindungen der Formel IIa und die Säuren als solche getrennt in die Reaktionslösung eingegeben werden. Hierzu eignen sich insbesondere wasserfreie leicht dosierbare anorganische oder organische Säuren, z. B. gasförmige anorganische Säuren wie Halogenwasserstoffsäuren, insbesondere Chlorwasserstoff, oder feste organische Säuren wie beispielsweise Oxalsäure oder p-Toluolsulfonsäure.

Es können jedoch auch zunächst aus den Verbindungen der Formel IIa und den Säuren Säureadditionssalze hergestellt werden, welche ein Äquivalent Säure enthalten, und diese dann in die Reaktion eingesetzt werden. Zur Salzbildung eignen sich alle Säuren, welche kristalline Salze bilden, insbesondere Halogenwasserstoffsäuren, Schwefelsäure, Phosphorsäure oder gegebenenfalls im Benzoling substituierte Benzolsulfonsäuren.

Die Hydrierung wird zweckmäßigerweise unter erhöhtem Druck beispielsweise bei einem Wasserstoffdruck von 5-100 bar, vorzugsweise 25-75, insbesondere 40-60 bar, durchgeführt. Als Katalysator wird handelsübliches Platinoxid verwendet, welches einen Platingehalt von 79-85 % besitzt und beispielsweise unter der Bezeichnung "Adamskatalysator" bekannt ist. Die Katalysatormenge kann je nach angewendetem Wasserstoffdruck variieren, beträgt zweckmäßig jedoch mindestens 2 g pro Mol der Verbindung der Formel IIa. Geeignet sind beispielsweise Mengen von 2-20, insbesondere 2-5 g Platinoxidkatalysator pro Mol Verbindung der Formel IIa. Die Reaktion kann bei Raumtemperatur oder erhöhter Temperatur beispielsweise erhöhten Temperaturen bis zu 80°C, vorzugsweise bei Temperaturen zwischen 30 und 80, insbesondere 40 und 60°C, durchgeführt werden. Die Reaktionszeit variiert je nach angewendetem Wasserstoffdruck, Temperatur und Katalysatormenge. Sie kann beispielsweise zwischen 2 und 5 Stunden liegen.

Das erfindungsgemäße Hydrierungsverfahren besitzt gegenüber den bisher zur Herstellung von Verbindungen der Formel I bekannten Reduktionsverfahren den Vorteil, daß die 3-Amino-1-benzoxepin-5(2H)-one der Formel IIa in nur einem Reaktionsschritt in 2,3,4,5-Tetrahydro-3-amino-1-benzoxepin-5-ole mit einem bereits so hohen Gehalt an cis-Epimeren überführt werden können, daß hieraus weitgehend transepimerenfreie cis-2,3,4,5-Tetrahydro-3-amino-1-benzoxepin-5-ole mit nur geringem Aufwand isoliert

werden können.

Die 3-Amino-1-benzoxepin-5(2H)-one der Formel IIa stellen vinyloge Amide dar. Es ist daher überraschend, daß bei der erfindungsgemäßen Hydrierung der Verbindungen der Formel IIa mit einem Platinoxidkatalysator unter den erfindungsgemäßen Reaktionsbedingungen eine Hydrierung der Doppelbindung der Verbindungen der Formel IIa und eine Reduktion der 5-Keto-Gruppe zur Hydroxygruppe ohne Spaltung des Moleküls stattfinden und die cis-2,3,4,5-Tetrahydro-3-amino-1-benzoxepin-5-ole der Formel I in guter Ausbeute erhalten werden. Es ist nämlich aus in der Literatur beschriebenen Untersuchungen über das Verhalten von vinylogen Amiden, d. h. β-Aminovinylketonen, bei katalytischer Hydrierung in Gegenwart verschiedener Hydrierungskatalysatoren, u.a. Platinoxid, bekannt, daß die katalytische Hydrierung entweder nur zu einer Hydrierung der Doppelbindung führt und die entsprechenden gesättigten Aminoketone erhalten werden, oder bei einer weitergehenden Reaktion eine Hydrierung der Doppelbindung und eine hydrogenolytische Spaltung des Moleküls zu Aminen und gesättigten Ketonen führt, siehe z. B. J. Amer. Chem. Soc. 68 (1946) 2009, 2010 oder Chem. Abstr. 49 (1955), 6090e.

Die Reduktion der Verbindungen der Formel II nach den erfindungsgemäßen Verfahren verläuft mit guten Ausbeuten und hoher Diastereoselektivität. So werden im allgemeinen Gesamtausbeuten an 2,3,4,5-Tetrahydro-3-amino-1-benzoxepin-5-olen zwischen 80 und 95 % erhalten. In dem erhaltenen Reduktionsprodukt liegt ein Verhältnis von cis- zu trans-Epimeren von mindestens 7 : 3 vor. Im allgemeinen liegt der Gehalt an cis-Epimeren in dem Gemisch zwischen ca. 70 und 85 %, insbesondere bei ca. 80 %.

Aus den nach den erfindungsgemäßen Reduktionsverfahren erhaltenen Gemischen aus 2,3,4,5-Tetrahydro-3-amino-1-benzoxepin-5-olen können die cis-Epimeren auf an sich bekannte Weise weiter angereichert und isoliert werden. So können z. B. die freien Basen oder deren Säureadditionssalze in geeigneten Lösungsmitteln, beispielsweise niederen Alkoholen, der fraktionierten Kristallisation unterworfen werden. Zweckmäßigerweise wird das Reaktionsgemisch zunächst in ein Säureadditionssalz überführt und dieses aus einem niederen Alkohol kristallisiert. Zur Salzbildung für die fraktionierte Kristallisation zur Anreicherung der cis-Epimeren haben sich insbesondere Maleinsäure, Chlorwasserstoffsäure und p-Toluolsulfonsäure als geeignet erwiesen. Als niedere Alkohole können Alkohole mit 1-4 Kohlenstoffstomen, vorzugsweise Isopropanol verwendet werden. Da in dem bei der erfindungsgemäßen Reduktion angefallenen Reduktionsprodukt die cis-Epimeren schon sehr stark angereichert sind, können schon durch nur ein- bis zweimalige Umkristallisation racemische cis-Verbindungen mit einem Gehalt von weniger als 5 % der entsprechenden trans-Epimeren erhalten werden. Gewünschtenfalls kann die Gewinnung der cis-Epimeren aus dem Gemisch auch durch chromatographische Trennung in an sich bekannter Weise beispielsweise an Kieselgel oder an Aluminiumoxid erfolgen. Als Elutionsmittel eignet sich beispielsweise Methylenchlorid mit einem Zusatz von einem Niederalkylalkohol, insbesondere Methanol, und einem niederen Mono- oder Dialkylamin oder Ammoniak, insbesondere wäßriger Ammoniaklösung. Auch hier erweist sich der hohe Gehalt an cis-Epimeren in dem Rohprodukt als sehr vorteilhaft da bei der chromatographischen Trennung weniger Mischfraktionen auftreten und auch Trennmaterialien mit geringem Trennvermögen eingesetzt werden können.

Die erhaltenen racemischen cis-2,3,4,5-Tetrahydro-3-amino-1-benzoxepin-5-ole der Formel I können gewünschtenfalls in an sich bekannter Weise durch Umsetzung mit geeigneten optisch aktiven Säuren und anschließende fraktionierte Kristallisation der gewonnenen Salze in ihre optisch aktiven Antipoden aufgetrennt werden. Aus erhaltenen Säureadditionssalzen der Verbindungen der Formel I können gewünschtenfalls die freien Basen auf an sich bekannte Weise freigesetzt werden und diese gewünschtenfalls wiederum in andere Säureadditionssalze mit pharmakologisch verträglich Säuren überführt werden.

Die nachfolgenden Beispiele sollen die Erfindung näher erläutern, ohne jedoch ihren Umfang einzuschränken.

**Beispiel 1:**

3,5-cis-3-Methylamino-2,3,4,5-tetrahydro-1-benzoxepin-5-ol.

A) Durch Einleiten von 10,7 g gasförmigem Chlorwasserstoff in 900 ml Methanol wird eine methanolische Salzsäurelösung hergestellt. In diese Lösung werden 56,7 g 3-Methylamino-1-benzoxepin-5(2H)-on unter Ausschluß von Luftfeuchtigkeit bei Raumtemperatur gelöst. Die Lösung wird mit einer Aufschwämmung von 0,75 g Platinoxidkatalysator (= Adams-Katalysator, Platingehalt 82 %) in Methanol versetzt. Das Reaktionsgemisch wird im Autoklaven auf 50°C erwärmt und mit Stickstoff gespült. Anschließend wird bei dieser Temperatur unter einem Wasserstoff-Druck von 50 Bar und Rühren mit einem Hubrührer 2,5 Stunden hydriert. Nach Beendigung der Hydrierung wird der Wasserstoff abgelassen, mit Stickstoff gespült und filtriert. Die filtrierte Lösung wird im Vakuum eingedampft. Als Rückstand werden 68,5 g Hydrierungsprodukt erhalten, welches ein rohes Gemisch der Hydrochloride von 3,5-cis- und 3,5-trans-3-Methylamino-2,3,4,5-tetrahydro-1-benzoxepin-5-ol darstellt.

Das erhaltene Hydrierungsgemisch wird durch Hochleistungsflüssigkeitschromatographie (= HPLC) analysiert. Hierbei wird die Substanzprobe an Kieselgel (reversed phase Material, Nucleosil-RP18-0,005 mm, Hersteller Machery und Nagel) getrennt und UV-spektometrisch (λ = 215 nm) detektiert. Als Elutionsmittel wird eine Lösungsmittelmischung mit einem linearen Gradienten aus einem Wasser/Phosphorsäure-Gemisch (1000/1) und einem Wasser/Acetonitril/Phosphorsäure-Gemisch (100/900/1) verwendet. Durch Auswertung

nach der Flächenprozent-Methode wird der Hydrochlorid-Gemisches bestimmt. Die Analyse ergibt, daß das Gemisch 76,8 % Hydrochlorid des 3,5-cis-3-Methylamino-2,3,4,5-tetrahydro-1-benzoxepin-5-ol und 18,6 % Hydrochlorid des 3,5-trans-3-Methylamino-2,3,4,5-tetrahydro-1-benzoxepin-5-ol enthält.

B) Isolierung des cis-Epimeren:

Aus dem nach Beispiel 1A erhaltenen Gemisch können das 3,5-cis-3-Methylamino-2,3,4,5-tetrahydro-1-benzoxepin-5-ol oder dessen Säureadditionssalze nach den folgenden Methoden erhalten werden:

$B_1$) 29 g des gemäß Beispiel 1A erhaltenen Hydrochloridgemisches werden in 50 ml Isopropanol gelöst und die Lösung wird bis zur beginnenden Kristallisation eingeengt und abkühlen gelassen. Nach dem Abkühlen wird das gebildete Kristallisat abfiltriert und dieser Umkristallisationsvorgang wird noch einmal wiederholt. Es werden 20 g 3,5-cis-3-Methylamino-2,3,4,5-tetrahydro-1-benzoxepin-5-ol-hydrochlorid erhalten, Schmelzpunkt 190°C.

$B_2$) 33 g des nach Beispiel 1A erhaltenen Hydrochlorid-Gemisches werden zur Freisetzung des entsprechenden Basengemisches mit 110 ml konzentrierter wäßriger Ammoniaklösung versetzt und das Reaktionsgemisch mit Methylenchlorid extrahiert. Die Methylenchloridphase wird über Natriumsulfat getrocknet und das Lösungsmittel abgedampft. Als Rückstand werden 28 g des Basengemisches erhalten.

Dieses Gemisch wird in 130 ml Isopropanol gelöst, die Lösung mit einer heißen Lösung von 20 g Maleinsäure in 60 ml Isopropanol versetzt und das erhaltene Gemisch bis zur beginnenden Kristallisation eingeengt. Nach dem Abkühlen wird das auskristallisierte maleinsaure Salz abfiltriert und aus 450 ml Isopropanol umkristallisiert, Schmelzpunkt 148 - 150°C.

Zur Freisetzung der Base wird das so erhaltene maleinsaure Salz der Titelverbindung mit wäßriger konzentrierter Ammoniaklösung versetzt und die freigesetzte Titelverbindung mit Methylenchlorid extrahiert. Nach Trocknen der Methylenchloridphase und Eindampfen des Lösungsmittels werden 21 g 3,5-cis-3-Methylamino-2,3,4,5-tetrahydro-1-benzoxepin-5-ol erhalten, Schmelzpunkt 100 - 103°C.

Zur Überführung in ihr Hydrochlorid werden 19,3 g der vorstehend erhaltenen Titelverbindung in Isopropanol gelöst und die Lösung mit 3,7 g gasförmigem Chlorwasserstoff versetzt. Anschließend wird bis zur beginnenden Kristallisation eingeengt und abkühlen gelassen. Das erhaltenen Hydrochlorid der Titelverbindung wird abfiltriert. Schmelzpunkt 190°C.

$B_3$) Aus dem nach Beispiel 1A erhaltenen Hydrochloridgemisch wird wie in Beispiel $1B_2$ beschrieben das Basengemisch freigesetzt. 4 g des erhaltenen Basengemisches werden an einer 110 g Kieselgel (Lichroprep$^R$ Si 60, Hersteller Merck) enthaltenden Chromatographiesäule (25 X 500 mm) unter einem Druck von ca. 2 bar unter Verwendung von Methylenchlorid/ Methanol/25 %-ige wäßrige Ammoniaklösung 100/20/2 als Elutionsmittel chromatographiert. Nach Eindampfen des Eluates erhält man 2,9 g 3,5-cis-3-Methylamino-2,3,4,5-tetrahydro-1-benzoxepin-5-ol mit einem Schmelzpunkt von 100 - 103°C.

**Beispiel 2:**

3,5-cis-3-Methylamino-2,3,4,5-tetrahydro-1-benzoxepin-5-ol.

A) 56,7 g 3-Methylamino-1-benzoxepin-5-(2H)-on und 57,1 g p-Toluolsulfonsäuremonohydrat werden in 900 ml Methanol gelöst. Nach Zusatz von 0,75 g Platinoxidkatalysator (=Adams-Katalysator) wird das Reaktionsgemisch wie in Beispiel 1A beschrieben hydriert und aufgearbeitet. Als Rückstand werden 110 g eines rohen Gemisches der p-toluolsulfonsauren Salze von 3,5-cis- und 3,5-trans-3-Methylamino-2,3,4,5-tetrahydro-1-benzoxepin-5-ol erhalten. Dieses Gemisch enthält nach Analyse durch HPLC (s. Beispiel 1A) neben 74,3 % des p-toluolsulfonsauren Salzes der Titelverbindung noch 15,5 % der entsprechenden 3,5-trans-epimeren Verbindung.

B) Isolierung des cis-Epimeren:

Das vorstehend erhaltene Gemisch der p-toluolsulfonsauren Salze wird in 200 ml Isopropanol gelöst und die Lösung wird bis zur beginnenden Kristallisation eingeengt und abkühlen gelassen. Nach dem Abkühlen wird das gebildete Kristallisat abfiltriert und nochmals in der vorbeschriebenen Weise aus Isopropanol umkristallisiert. Es wird reines 3,5-cis-3-Methylamino-2,3,4,5-tetrahydro-1-benzoxepin-5-ol-p-toluolsulfonat mit einem Schmelzpunkt von 163 -165°C erhalten.

Gewünschtenfalls können aus dem in Beispiel 2A erhaltenen Gemisch der p-toluolsulfonsauren Salze die Titelverbindung und ihr Hydrochlorid analog den in Beispiel $1B_2$ und $1B_3$ beschriebenen Methoden gewonnen werden.

**Beispiel 3:**

3,5-cis-3-Methylamino-2,3,4,5-tetrahydro-1-benzoxepin-5-ol.

A) Zur Überführung in das Hydrochlorid werden 19 g 3-Methylamino-1-benzoxepin-5(2H)-on in 130 ml Methanol suspendiert und unter Rühren gasförmiger Chlorwasserstoff in die Suspension eingeleitet. Hierbei geht das Amin unter Erwärmung in Lösung. Anschließend werden ca. 40 ml Methanol aus der Lösung abdestilliert. Die Lösung wird zur Kristallisation des gebildeten Hydrochlorids stehengelassen und die

gebildeten Kristalle werden abfiltriert Es werden 22 g 3-Methylamino-1-benzoxepin-5(2H)-on-hydrochlorid erhalten, welches ohne weitere Reinigung in die nachfolgende Hydrierung eingesetzt wird.

B) 22 g 3-Methylamino-1-benzoxepin-5(2H)-on-hydrochlorid werden in Gegenwart von 0,2 g Adams-Katalysator in 150 ml Methanol bei 40°C unter einem Wasserstoffdruck von 50 bar während 3,5 Stunden hydriert und das Reaktionsgemisch wird wie in Beispiel 1A beschrieben aufgearbeitet. Das als Rückstand erhaltene Hydrierungsprodukt enthält nach Analyse durch HPLC wie in Beispiel 1A beschrieben 77,1 % des Hydrochlorids der Titelverbindung neben 18,2 % der entsprechenden 3,5-trans-Verbindung.

Die Titelverbindung (Fp.: 100 - 103°C) und ihr Hydrochlorid (Fp.: 190°C) können aus dem Gemisch wie in Beispiel 1B, beschrieben gewonnen werden.

**Beispiel 4:**

3,5-cis-3-Methylamino-2,3,4,5-tetrahydro-1-benzoxepin-5-ol.

3,5-Cis-Methylamino-2,3,4,5-tetrahydro-1-benzoxepin-5-ol wird analog zu den in Beispielen 1A, 2A und 3A+B beschriebenen Methoden durch Hydrierung von 3-Methylamino-1-benzoxepin-5(2H)-on in Gegenwart von Adams-Katalysator auch unter den in der nachstehenden Tabelle I angegebenen Reaktionsbedingungen erhalten. In der Tabelle werden außer den Reaktionsbedingungen noch die Gesamtausbeuten an 3-Methylamino-2,3,4,5-tetrahydro-1-benzoxepin-5-olen sowie das durch HPLC nach der in Beispiel 1A angegebenen Methode bestimmte Verhältnis von 3,5-cis zu 3,5-trans-Verbindungen in dem Hydrierungprodukt angegeben.

**Tabelle I**

| Beispiel Nr. | Ausgangsprod. eingesetzt als *** | Säure *** Zusatz (Äquivalent *) | Lösungsmittel (ml *) | $PtO_2$ menge g * | $H_2$-Druck bar | Reakt.-Temp. °C | Reakt.-zeit h | Gesamtausbeute % | cis/trans % |
|---|---|---|---|---|---|---|---|---|---|
| 4a | Ba | HCl (0,95) | $CH_3OH$ (3024) | 2,46 | 100 | 40 | 2,5 | 78 | 80/20 |
| 4b | Ba | HCl (1,05) | $CH_3OH$ (3024) | 2,46 | 100 | 40 | 2,5 | 79 | 79/21 |
| 4c | Cl | - | $CH_3OH$ (2800) | 16 | 6 | 50 | 2,5 | 81 | 72/28 |
| 4d | Su | - | $CH_3OH$ (2800) | 16 | 6 | 50 | 2,5 | 81 | 80/20 |
| 4e | Ph | - | $CH_3OH$ (2800) | 16 | 6 | 50 | 2,5 | 80 | 81/19 |
| 4f | Ba | Cyc (1,00) | $CH_3OH$ (3024) | 2,46 | 50 | 50 | 2,5 | 83 | 77/23 |
| 4g | Ba | Oxa (1,00) | $CH_3OH$ (2800) | 16 | 6 | 50 | 2,5 | 85 | 82/18 |

* bezogen auf 1 Mol 3-Methylamino-1-benzoxepin-5(2H)-on
**Cl = Hydrochlorid, Su = Sulfat, Ph = Phosphat, Ba = Base
***HCl = Chlorwasserstoff, Cyc = Cyclohexylaminosulfonsäure, Oxa = Oxalsäure

**Beispiel 5:**

3,5-cis-7-Clor-3-methylamino-2,3,4,5-tetrahydro-1-benzoxepin-5-ol.

Die Titelverbindung wird durch Hydrierung von 7-Chlor-3-methylamino-1-benzoxepin-5(2H)-on Wasserstoff nach der in Beispiel 3A+B beschriebenen Methode erhalten. Die Hydrierung erfolgt bei Raumtemperatur und einem Wasserstoffdruck von 100 bar. Die Reaktionszeit beträgt 4 Stunden. In dem Hydrierungsprodukt (Gesamtausbeute an 7-Chlor-3-methylamino-2,3,4,5-tetrahydro-1-benzoxepin-5-ol-hydrochloriden = 73 %) beträgt gemäß HPLC-Analyse (siehe Beispiel 1A) das Verhältnis von 3,5-cis-Verbindung zu 3,5-trans-Verbindung 88 : 12. Aus dem erhaltenen Gemisch wird das Hydrochlorid der Titelverbindung nach der in Beispiel $1B_1$ beschriebenen Methode gewonnen, Schmelzpunkt: 172 - 173 °C.

**Beispiel 6:**

3,5-cis-3-Äthylamino-2,3,4,5-tetrahydro-1-benzoxepin-5-ol.

Die Titelverbindung wird durch Hydrierung von 3-Äthylamino-1-benzoxepin-5(2H)-on mit Wasserstoff nach der in Beispiel 3A+B beschriebenen Methode hergestellt. Die Hydrierung erfolgt bei 50°C und einem Wasserstoffdruck von 6 bar. Die Reaktionszeit beträgt 2,5 Stunden. In dem Hydrierungsprodukt (Gesamtausbeute an 3-Äthylamino-2,3,4,5-tetrahydro-1-benzoxepin-5-ol-hydrochloriden = 79 %) beträgt das Verhältnis von 3,5-cis-Verbindung zu 3,5-trans-Verbindung gemäß HPLC-Analyse (siehe Beispiel 1A) 79 : 21. Aus dem erhaltenen Gemisch wird analog der in Beispiel $1B_2$ beschriebenen Methode das Cyclohexylsulfonat der Titelverbindung gewonnen, Schmelzpunkt: 150°C.

**Beispiel 7:**

3,5-cis-7-Methyl-3-methylamino-2,3,4,5-tetrahydro-1-benzoxepin-5-ol.

Die Titelverbindung wird durch Hydrierung von 7-Methyl-3-methylamino-1-benzoxepin-5(2H)-on mit Wasserstoff nach der in Beispiel 3A+B beschriehenen Methode erhalten. Die Hydrierung erfolgt bei einer Temperatur von 50°C und einem Wasserstoffdruck von 6 bar. Die Reaktionszeit beträgt 2,5 Stunden. In dem Hydrierungsprodukt (Gesamtausbeute an 7-Methyl-3-methylamino-2,3,4,5-tetrahydro-1-benzoxepin-5-ol-hydrochloriden =76 %) beträgt gemäß HPLC-Analyse (siehe Beispiel 1A) das Verhältnis von 3,5-cis-Verbindung zu 3,5-trans-Verbindung 78 : 22. Aus dem erhaltenen Gemisch wird das Hydrochlorid der Titelverbindung nach der in Beispiel $1B_1$ beschriebenen Methode gewonnen, Schmelzpunkt 199 - 200°C.

**Beispiel 8:**

3,5-cis-3-Methylamino-2,3,4,5-tetrahydro-1-benzoxepin-5-ol.

Zu einem Gemisch von 189 g 3-Methylamino-1-benzoxepin-5(2H)-on und 174 g Boran/tert. Butylamin-Komplex in 500 ml Toluol werden 80 ml Essigsäure gegeben und das Reaktionsgemisch wird 45 Minuten gerührt. Die Temperatur steigt dabei auf 36°C an. Anschließend werden innerhalb 30 Minuten 100 ml Essigsäure eingetropft. Die Temperatur steigt auf 60° an. Es wird auf 50°C abgekühlt und 30 Minuten weitergerührt. Anschließend werden weitere 60 ml Essigsäure zugefügt und durch Erwärmen die Temperatur für zwei weitere Stunden auf 50-60 °C gehalten. Ohne vorheriges Abkühlen wird das Reaktionsgemisch in eine Mischung aus 1000 g zerstoßenem Eis und 250 ml konzentrierter Salzsäure so eingerührt, daß die Temperatur 25°C nicht übersteigt. Die organische Phase wird abgetrennt und dreimal mit je 90 ml Wasser und 10 ml konzentrierter Salzsäure nachgewaschen Die wäßrige Phase wird durch Zugabe von 540 ml konzentriertem Ammoniak alkalisch gestellt und dreimal mit je 500 ml und viermal mit je 200 ml Methylenchlorid extrahiert. Die vereinigten Methylenchloridphasen werden über Natriumsulfat getrocknet, filtriert und das Lösungsmittel unter vermindertem Druck abdestilliert. Es werden 183 g Rohprodukt erhalten, welches ein Gemisch aus 3,5-cis-3-Methylamino-2,3,4,5-tetrahydro-1-benzoxepin-5-ol und der entsprechenden 3,5-trans-Verbindung darstellt. Gemäß HPLC-Analyse nach der in Beispiel 1A beschriebenen Methode enthält das Gemisch 81,8 % 3,5-cis-3-Methylamino-2,3,4,5-tetrahydro-1-benzoxepin-5-ol und 18,2 % der entsprechenden 3,5-trans-Verbindung.

Aus diesem Gemisch werden die Titelverbindung (Fp.: 100-103°C) oder deren Hydrochlorid (Fp.: 190°C) analog den in Beispiel $1B_1$ und $1B_2$ beschriebenen Methoden erhalten.

**Beispiel 9:**

3,5-cis-3-Methylamino-2,3,4,5-tetrahydro-1-benzoxepin-5-ol.

Die Titelverbindung wird analog der in Beispiel 8 beschriebenen Methode durch Reduktion von 3-Methylamino-1-benzoxepin-5(2H)-on mit einem Boran/Amin-Komplex auch unter den in dem nachstehenden Tabelle II angegebenen Reaktionsbedingungen erhalten. Neben den Reaktionsbedingungen werden in der Tabelle die Gesamtausbeuten an 3-Methylamino-2,3,4,5-tetrahydro-1-benzoxepin-5-olen sowie das mittels HPLC nach der in Beispiel 1A beschriebenen Methode bestimmte Verhältnis von 3,5-cis zu 3,5-trans-Verbindung angegeben.

**Tabelle II**

| Beispiel Nr. | BH3/Amin-komplex Amin Komponente | Komplex Menge mol * | Lösungsmittelgemisch ** aprot. Lösungsm. / Säure | (Volumenanteile) | Reakt. Temp. °C | Reakt. Zeit h | Gesamt-ausbeute % | cis/trans % |
|---|---|---|---|---|---|---|---|---|
| 9a | $HN(CH_3)_2$ | 2 | Tol/Acet | (50 : 24) | 60 | 4 | 85 | 65/35 |
| 9b | $H_2N\text{-}C(CH_3)_3$ | 2 | Tol/Acet | (20 : 3) | RT*** | 50 | 80 | 85/15 |
| 9c | $H_2N\text{-}C(CH_3)_3$ | 2 | $CH_2Cl_2$/Acet | (20 : 2,4) | RT*** | 48 | 90 | 90/10 |
| 9d | $H_2N\text{-}C(CH_3)_3$ | 2 | Tol/Acet | (20 : 2,4) | 45 | 3 | 90 | 88/12 |
| 9e | $H_2N\text{-}C(CH_3)_3$ | 2 | $CH_2Cl_2$/Acet | (20 : 2,4) | 45 | 6 | 89 | 89/11 |
| 9f | $H_2N\text{-}C(CH_3)_3$ | 3 | Tol | | 100 | 48 | 65 | 64/36 |

* bezogen auf 1 Mol 3-Methylamino-1-benzoxepin-5(2H)-on
** Tol = Toluol, $CH_2Cl_2$ = Methylenchlorid, Acet = Eisessig
*** RT = Raumtemperatur

**Beispiel 10:**

3,5-cis-7-Chlor-3-methylamino-2,3,4,5-tetrahydro-1-benzoxepin-5-ol.

Die Titelverbindung wird durch Reduktion von 7-Chlor-3-methylamino-1-benzoxepin-5(2H)-on mit Boran/tert. Butylamin nach der in Beispiel 8 beschriebenen Methode erhalten. In dem Reduktionsprodukt (Gesamtausbeute an 7-Chlor-3-methyl-amino-2,3,4,5-tetrahydro-1-benzoxepin-5-olen 70 %) beträgt das Verhältnis von 3,5-cis-Verbindung zu 3,5-trans-Verbindung gemäß HPLC-Analyse nach der in Beispiel 1A beschriebenen Methode 98 : 2. Aus dem erhaltenen Gemisch wird das Hydrochlorid der Titelverbindung nach der in Beispiel $1B_2$ beschriebenen Methode gewonnen, Schmelzpunkt: 172 - 173 °C.

**Beispiel 11:**

3,5-cis-3-Diäthylamino-2,3,4,5-tetrahydro-1 benzoxepin-5-ol.

Die Titelverbindung wird durch Reduktion von 3-Diäthylamino-1-benzoxepin-5(2H)-on mit Boran/tert. Butylamin nach der in Beispiel 8 beschriebenen Methode hergestellt. In dem Reduktionsprodukt (Gesamtausbeute an 3-Diäthylamino-2-3,4,5-tetrahydro-1-benzoxepin-5(2H)-olen = 91 %) beträgt das Verhältnis von 3,5-cis-Verbindung zu 3,5-trans-Verbindung gemäß HPCL-Analyse nach der in Beispiel 1A beschrieben Methode 67 : 33. Aus dem erhaltenen Gemisch wird analog der in Beispiel $1B_2$ beschriebenen Methode das p-Toloulsulfonat der Titelverbindung gewonnen, Schmelzpunkt: 185-187° C.

**Beispiel 12:**

3,5-cis-7-Methyl-3-methylamino-2,3,4,5-tetrahydro-1-benzoxepin-5-ol.

Die Titelverbindung wird durch Reduktion von 7-Methyl-3-methylamino-1-benzoxepin-5(2H)-on mit Boran/tert. Butylamin nach der in Beispiel 8 beschriebenen Methode erhalten. In dem Reduktionsprodukt (Gesamtausbeute an 7-Methyl-3-methylamino-2,3,4,5-tetrahydro-1-benzoxepin-5-olen = 73 %) beträgt das Verhältnis von 3,5-cis-Verbindung zu 3,5-trans-Verbindung gemäß HPLC-Analyse nach der in Beispiel 1A beschriebenen Methode 96 : 4. Aus dem erhaltenen Gemisch wird das Hydrochlorid der Titelverbindung nach der in Beispiel $1B_2$ beschriebenen Methode gewonnen, Schmelzpunkt: 199 - 200° C.

**Patentansprüche**

1. Verfahren zur Herstellung von cis-2,3,4,5-Tetrahydro-3-amino-1-benzoxepin-5-olen der allgemeinen Formel I

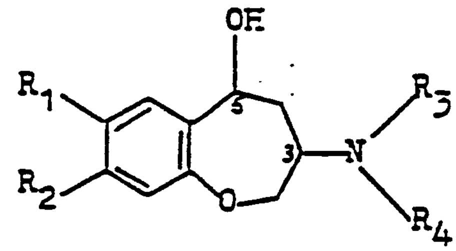

I

worin die Substituenten in 3- und 5-Stellung zueinander cis-ständig sind und
$R_1$ Wasserstoff, Halogen oder Alkyl mit 1-4 Kohlenstoffatomen bedeutet,
$R_2$ Wasserstoff, Halogen oder Alkyl mit 1-4 Kohlenstoffatomen bedeutet,
$R_3$ Wasserstoff oder Alkyl mit 1-5 Kohlenstoffatomen bedeutet, und
$R_4$ Wasserstoff oder Alkyl mit 1-5 Kohlenstoffatomen bedeutet,
und deren Säureadditionssalzen, dadurch gekennzeichnet, daß man 3-Amino-1-benzoxepin-5-(2H)-one der allgemeinen Formel II

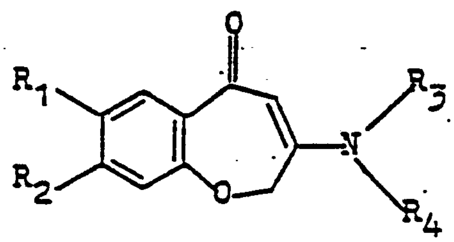

II

worin $R_1$, $R_2$, $R_3$ und $R_4$ obige Bedeutung besitzen, einstufig diastereoselektiv zu einem Gemisch von 2,3,4,5-Tetrahydro-3-amino-1-benzoxepin-5-olen mit einem überwiegenden Anteil an cis-Verbindungen der allgemeinen Formel I reduziert, indem man

a) Verbindungen der allgemeinen Formel II mit einem Boran/Amin-Komplex in einem 0-50 Vol.-% einer flüssigen, niederen aliphatischen Carbonsäure enthaltenden, aprotischen organischen Lösungsmittel umsetzt, oder

b) Verbindungen der allgemeinen Formel IIa

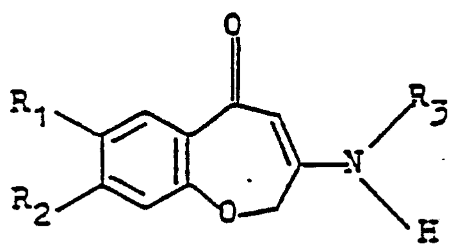

IIa

worin $R_1$, $R_2$, und $R_3$ obige Bedeutung besitzen, mit Wasserstoff in Gegenwart eines Platinoxidkatalysators in einem organischen protischen, polaren Lösungsmittel in Gegenwart einer äquivalenten Menge einer unter den Reaktionsbedingungen stabilen Säure hydriert,

und aus dem erhaltenen Reduktionsprodukt die cis-2,3,4,5-Tetrahydro-3-amino-1-benzoxepin-5-ole in Form ihrer Säureadditionssalze oder der freien Basen isoliert und gewünschtenfalls die Säureadditionssalze in die freien Basen überführt oder die freien Basen in ihre Säureadditionssalze überführt.

2. Verfahren nach Anspruch 1, daurch gekennzeichnet, daß man als Verbindung der allgemeinen Formel II oder II a das 3-Methylamino-1-benzoxepin-5(2H)-on einsetzt.

3. Verfahren nach Anspruch 1, dadurch gekennzeichnet, daß man in der Verfahrensvariante a) als Boran/Amin-Komplex einen Komplex aus Boran und einem Monoalkylamin mit 1-4 Kohlenstoffatomen oder einem Dialkylamin, worin eine Alkylgruppe 1-2 und die andere Alkylgruppe 1-4 Kohlenstoffatome enthält,

einsetzt.

4. Verfahren nach Anspruch 3, dadurch gekennzeichnet, daß man als Boran/Amin-Komplex einen Boran/tert. Butylamin-Komplex einsetzt.

5. Verfahren nach Anspruch 1, dadurch gekennzeichnet, daß man die Umsetzung gemäß Verfahrensvariante a) in einem Gemisch aus einem aprotischen organischen Lösungsmittel und einer flüssigen niederen aliphatischen Carbonsäure im Volverhältnis 9 : 1 bis 1 : 1 durchführt.

6. Verfahren nach Anspruch 5, dadurch gekennzeichnet, daß man als flüssige niedere aliphatische Carbonsäure eine Alkancarbonsäure mit 2-3 Kohlenstoffatomen einsetzt.

7. Verfahren nach Anspruch 1, dadurch gekennzeichnet, daß man in der Verfahrensvariante b) als Säuren Halogenwasserstoffsäuren oder Benzolsulfonsäuren, worin der Benzolring unsubstituiert oder durch niederes Alkyl oder Halogen substituiert ist, einsetzt.

8. Verfahren nach Anspruch 1 oder 7, dadurch gekennzeichnet, daß man in der Verfahrensvariante b) die Verbindung der Formel II a mit der Säure zu einem 1 Äquivalent Säure enthaltenden Säureadditionssalze umsetzt und dieses zur Hydrierung einsetzt.

9. Verfahren nach Anspruch 8, dadurch gekennzeichnet, daß man als Säure Clorwasserstoffsäure einsetzt.

10. Verfahren nach Anspruch 7, dadurch gekennzeichnet, daß man die Hydrierung in Gegenwart von 2-5 g Platinoxidkatalysator pro Mol der Verbindung der Formel IIa bei einem Wasserstoffdruck von 40-60 bar in einem niederen Alkohol durchführt.

**Claims**

1. Method for producing cis-2,3,4,5-tetrahydro-3-amino-1-benzoxepin-5-ols the general Formula I,

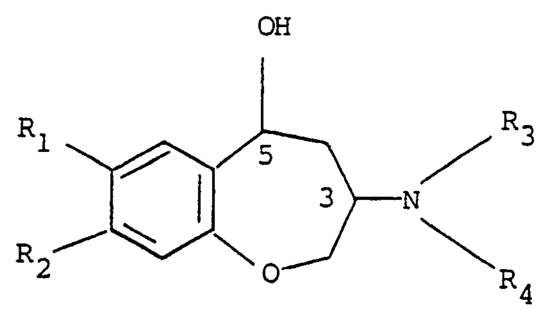

I

wherein the substituents in the 3 and 5 positions are in cis-relationship to each other and

$R_1$ represents hydrogen, halogen or alkyl with 1-4 carbon atoms,

$R_2$ represents hydrogen, halogen or alkyl with 1-4 carbon atoms,

$R_3$ represents hydrogen or alkyl with 1-5 carbon atoms, and

$R_4$ represents hydrogen or alkyl with 1-5 carbon atoms,

and their acid addition salts, characterised in that 3-amino-1-benzoxepin-5-(2H)-ones of the general Formula II,

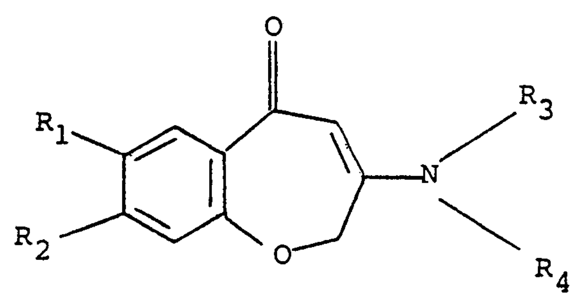

II

wherein $R_1$, $R_2$, $R_3$ and $R_4$ have the above meanings, are reduced diastereoselectively in a single step to a mixture of 2,3,4,5-tetrahydro-3-amino-1-benzoxepin-5-ols with a predominant fraction of the cis compounds of the general Formula I, by

a) reacting compounds of the general Formula II with a borane/amine complex in an aprotic organic solvent containing 0-50 % vol. of a liquid lower aliphatic carboxylic acid, or

b) hydrogenating compounds of the general Formula IIa,

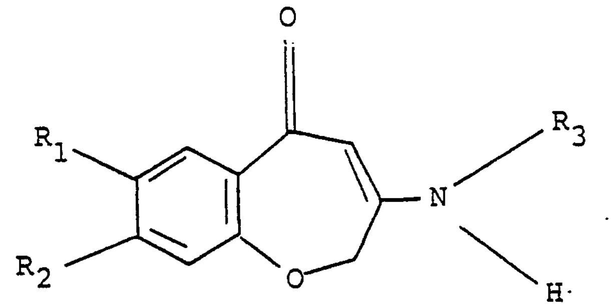

IIa

wherein $R_1$, $R_2$ and $R_3$ have the above meaning, with hydrogen in the presence of a platinum oxide catalyst in an organic protic polar solvent in the presence of an equivalent quantity of an acid which is stable under the reaction conditions,

and isolating the cis-2,3,4,5-tetrahydro-3-amino-1-benzoxepin-5-ols from the resulting reaction product in the form of their acid addition salts or the free bases and if desired converting the acid addition salts into the free bases or converting the free bases into their acide addition saltes.

2. Method according to Claim 1, characterised in that 3-methylamino-1-benzoxepin-5-(2H)-one is used as the compound of the general Formula II or IIa.

3. Method according to Claim 1, characterised in that in process variant a) a complex of borane and a monoalkylamine with 1-4 carbon atoms or a dialkylamine, wherein one alkyl group contains 1-2 and the other alkyl group 1-4 carbon atoms, is used as the borane/amine complex.

4. Method according to Claim 3, characterised in that a borane/tert. butylamine complex is used as the borane/amine complex.

5. Method according to Claim 1, characterised in that the reaction according to process variant a) is carried out in a mixture of an aprotic organic solvent and a liquid lower aliphatic carboxylic acid in the volume ratio 9 : 1 to 1 : 1.

6. Method according to Claim 5, characterised in that an alkane carboxylic acid with 2-3 carbon atoms is used as the liquid lower aliphatic carboxylic acid.

7. Method according to Claim 1, characterised in that in process variant b) halogen hydracids or benzene sulphonic acids, wherein the benzene ring is unsubstituted or substituted by lower alkyl or halogen, are used as acids.

8. Method according to Claim 1, characterised in that in process variant b) the compound of Formula IIa is reacted with the acid to an acid addition salt containing 1 equivalent acid and the latter is used for hydrogenation.

9. Method according to Claim 8, characterised in that hydrochloric acid is used as the acid.

10. Method according to Claim 7, characterised in that the hydrogenation is carried out in the presence of 2-5 g platinum oxide catalyst per mole of the compound of Formula IIa at a hydrogen pressure of 40-60 bar in a lower alcohol.

## Revendications

1. Procédé ce préparation de cis-2,3,4,5-tétrahydro-3-amino-1-benzoxepine-5-ols de formule générale 1

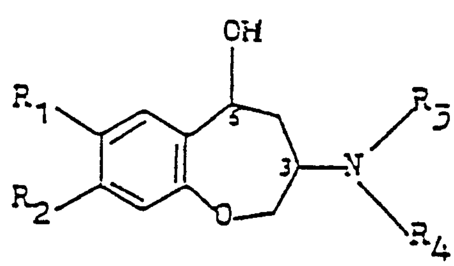

I

dans laquelle les substituants en position 3 et 5 sont l'un par rapport à l'autre en cis, et

$R_1$ représente un atome d'hydrogène ou d'halogène ou un groupe alkyle ayant 1 à 4 atomes de carbone,
$R_2$ représente un atome d'hydrogène ou d'halogène ou un groupe alkyle ayant 1 à 4 atomes de carbone,
$R_3$ représente un atome d'hydrogène ou un groupe alkyle ayant 1 à 5 atomes de carbone, et
$R_4$ représente un atome d'hydrogène ou un groupe alkyle ayant 1 à 5 atomes de carbone,

et de leurs sels d'addition d'acides procédé caractérisé en ce qu'on réduit diastereoselectivement des 3-amino-1-benzoxepine-5-(2H)-ones de formule générale II

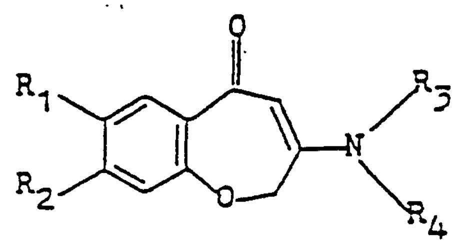

II

dans laquelle $R_1$, $R_2$, $R_3$ et $R_4$ ont le sens ci-dessus en une seule étape, pour obtenir un mélange de 2,3,4,5-tétrahydro-3-amino-1-benzoxepine-5-ols comportant une proportion prédominante de composés cis de formule générale I,

a) en faisant réagir des composés ce formule générale II avec un complexe borane/amine dans un solvant organique aprotique contenant 0 à 50 % en volume d'un acide carboxylique aliphatique inférieur liquide, ou

b) en hydrogénant des composés de fornule générale IIa

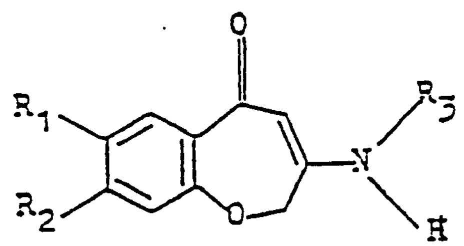

IIa

(dans laquelle $R_1$, $R_2$ et $R_3$ ont le sens ci-dessus) avec ce l'hydrogène en présence d'un catalyseur à l'oxyde de platine dans un solvant organique protique polaire, en présence d'une quantité équivalente d'un acide stable dans les conditions de réduction,

et en isolant, à partir du produit de réduction obtenu, les cis-2,3,4,5-tétrahydro-3-amino-benzoxepine-5-ols sous forme de leurs sels d'addition d'acides ou sous forme des bases libres et en transformant, si on le souhaite, les sels d'addition d'acides en les bases libres ou les bases libres en leurs sels d'addition d'acides.

2. Procédé selon la revendication 1, caractérisé en ce qu'on utilise la 3-méthylamino-1-benzoxépine-5(2H)-one comme composé de formule II ou IIa.

3. Procédé selon la revendication 1 , caractérisé en ce que, dans la variante opératoire a), on utilise comme complexe borane/amine un complexe de borane et d'une monoalkylamine ayant 1 à 4 atomes de carbone ou d'une dialkylamine dans lequel un groupe alkyle contient 1 ou 2 atomes de carbone et l'autre groupe alkyle 1 à 4 atomes de carbone.

4. Procédé selon la revendication 3, caractérisé en ce qu'on utilise comme complexe borane/amine un complexe borane/tert.-butylamine.

5. Procédé selon la revendication 1, caractérisé en ce qu'on conduit la réaction selon la variante opératoire a) dans un mélange, selon un rapport en volume de 9 : 1 à 1 : 1, d'un solvant organique aprotique et d'un acide carboxylique aliphatique inférieur liquide.

6. Procédé selon la revendication 5, caractérisé en ce qu'on utilise comme acide carboxylique aliphatique inférieur liquide un acide alcanecarboxylique comportant 2 ou 3 atomes de carbone.

7. Procédé selon la revendication 1, caractérisé en ce que, dans la variante opératoire b), on utilise comme acides des hydracides halogénés ou ces acides benzènesulfoniques, le noyau benzénique étant non substitué ou étant substitué par un groupe alkyle inférieur ou par de l'halogène.

8. Procédé selon la revendication 1 ou 7 , caractérisé en ce que, dans la variante opératoire b), on fait réagir le composé de formule II a avec l'acide pour obtenir un sel d'addition d'acide contenant l'équivalent d'acide et l'on utilise ce sel pour une hydrogénation.

9. Procédé selon la revendication 8, caractérisé en ce qu'on utilise comme acide l'acide chlorhydrique.

10. Procédé selon la revendication 7, caractérisé en ce qu'on conduit l'hydrogénation en opérant en présence de 2 à 5 g d'un catalyseur à l'oxyde de platine par mole de composé de formule IIa sous une pression d'hydrogène de 40 à 60 bars, dans un alcool inférieur.